# EUROPEAN PATENT SPECIFICATION

(11) **EP 0 461 225 B1**
(45) Date of publication and mention of the grant of the patent: **18.03.1998**
(21) Application number: 91901077.7
(22) Date of filing: 07.12.1990
(51) Int. Cl.: A61K 31/52, A61K 31/505, A61K 31/70

(54) **CIRCUMVENTION OF HUMAN TUMOR DRUG RESISTANCE**
UMGEHUNG DER RESISTENZ GEGEN ANTITUMORSTOFFE BEI MENSCHEN
CIRCONVENTION DE LA RESISTANCE HUMAINE AUX MEDICAMENTS DE LA TUMEUR

(30) Priority: 08.12.1989 US 447593
(43) Date of publication of application: 18.12.1991
(73) Proprietor: CITY OF HOPE, Duarte California 91010-0269 (US)
(72) Inventor: SCANLON, Kevin, J., Pasadena, CA 91107 (US); SOWERS, Lawrence, C., Duarte, CA 91010 (US)
(74) Representative: Hall, Marina
(86) International application number: US9007155
(87) International publication number: WO9108743

(56) References cited:
- WO-A-89/02733
- CANCER COMMUNICATIONS, vol. 1, no. 4, October 1989, pages 269-275, Pergamon Press plc, US; K.J. SCANLON et al.: "Overexpression of DNA replication and repair enzymes in cisplatin-resistant human colon carcinoma HCT8 cells and circumvention by azidothymidine"
- MOLECULAR PHARMACOLOGY, vol. 36, no. 3, September 1989, pages 360-365, The American Society for Pharmacology and Experimental Therapeutics; B. GOZ et al.: "1-beta-D-arabinofuranosylcytosine enhancement of resistance to several antineoplastic drugs in mammalian tissue culture cells"
- CANCER DRUG RESISTANCE, vol. 223, 1986, pages 187-202, Alan R. Liss, Inc.; Y.M. RUSTUM et al.: "Approaches to overcome in vivo anti-cancer drug resistance"
- THE BRITISH JOURNAL OF CANCER, vol. 45, no. 4, April 1982, pages 559-564; V.J. RICHARDSON et al.: "Liposomally trapped AraCTP to overcome AraC resistance in a murine lymphoma in vitro"
- Anti Cancer Drug Design, Volume 1 issued 1986 J.A. DOUBLE "Nucleoside Analogues 5. Molecular Combination of Anti-Cancer Drugs: Activity 5 Fluorouracil/ Nitrosourea Combinations against Mouse Colon Tumors" See pages 133-139.
- Journal of Heterocyclic Chemistry, Volume 24, issued 1987, S.H. CHU, "Synthesis of Aminomethyl and Amino Analogues of 5-Benzylacyclouridine and 5- Benzyloxybenzylacyclouridine" See pages 989-995.

## Description

The invention relates to the prevention, circumvention or amelioration of resistance of viable human tumour cells to DNA damaging chemotherapeutic agents, to methotrexate or to radiation.

### BACKGROUND OF THE INVENTION

The efficacy for cancer therapy of radiation and drugs, such as cisplatin, is often limited by the development of resistance. Biochemistry and tissue culture studies indicate that such resistance is a function of the capacity of cancer cells to repair damaged DNA. Parent application Serial No. 234,096, in accord with various published papers, demonstrates enhanced expression of DNA repair enzymes by DNA resistant phenotypes of certain human carcinoma cell lines. See, e.g., Lai, G.M., et al., Biochem.Pharmacol. 37:4597-4600 (1988); Hospers, G.A.P., et al., Cancer Res. 48:6803-6807 (1988); Masuda, H., et al., Cancer Res. 48:5713-5716 (1988); Kraker, A., et al., Cancer Lett. 38:307-314 (1988); Scanlon, K.J., et al., Cancer Investigation 7:563-589 (1989) (in press-incorporated herein by reference); and Scanlon, K.J., et al., Anticancer Res. 9:1301-1312 (1989) (incorporated herein by reference). See also, Murray, D., et al., Cancer Res. 45:6446-6452 (1985) and Miller, M.R., et al., J.Biol.Chem. 257:10204-10209 (1982).

AZT and various other nucleoside analogs are selective inhibitors of retroviral reverse transcriptases and of human DNA polymerases α, β and γ. See White, E.L., Biochem. and Biophys. Res. Comm. 161:393-398 (1989); Swinnen, L.J., et al., Cancer Res. 49:1383-1389 (1989); Ahnstrom, G., Biochimica et Biophysica Acta 1007:357-358 (1989); Elion, G.B., Science 244:41-47 (1989); Lin, T.-S., et al., J.Med.Chem. 32:1891-1895 (1989); Liu, S.-Y., et al., Cancer Res. 49:1366-1370 (1989); Ono, K., et al., Mol. Pharmacol. 35:578-583 (1989); Yarchoan, R., et al., New Eng.J.Med. 321:726-738 (1989) and United States patent 4,861,759.

### SUMMARY OF THE INVENTION

This invention relates to the circumvention or amelioration of resistance attributable to an enhanced DNA repair capacity of cancer cells damaged by radiation or chemotherapy.

United States application Serial No. 234,096 (= EP-A-0408675) states that properties that render human cancer cells resistant to chemotherapeutic drugs include enhanced expression of DNA repair enzymes and that new properties appear to enhance the sensitivity of the same cells to other drugs. For example, human leukemia cells resistant to cisplatin evidence enhanced sensitivity to dideoxy cytidine.

This phenomenon is observed because dideoxy cytidine (ddC), AZT and other nucleoside analogs are metabolized by cellular enzymes (kinases) to the triphosphates which serve as suicide substrates for DNA repair and replication enzymes, including among others DNA polymerase β. The consequent inhibition of DNA repair and replication enhances the cytotoxicity of radiation, DNA damaging chemotherapeutic agents and methotrexate in the resistant but not necessarily in the parental cell line.

The invention includes the discovery that the administration of certain nucleoside analogs, either alone or in combination with a DNA damaging agent such as radiation, cisplatin or methotrexate, avoids, ameliorates or circumvents human tumor cell resistance to such agents. Utilization of this discovery in the treatment of human patients is an important aspect of this invention.

When used in combination, sequential administration of the DNA damaging agent followed by administration of the nucleoside analog yields the desired result. The DNA damaging agent may also be administered simultaneously with the nucleoside analog, for example, by concurrent intravenous injection or by the use of liposomes in which the DNA damaging agent and nucleoside analog are co-entrapped.

Another aspect of the invention includes a method for screening nucleoside analogs and derivatives thereof for utility as antitumor agents.

### DETAILED DESCRIPTION OF THE INVENTION

The invention, therefore, relates to the use of a nucleoside analogue of Formula I or Formula II: 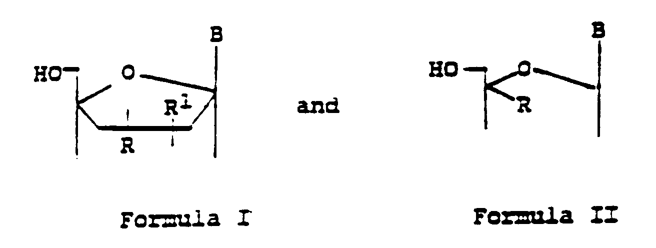 wherein:
B is adenine, thymine, cytosine, guanine, inosine or a heterocyclic compound derived therefrom, for example 5-methylcytosine; and
R and R¹ are, independently, halogen, azide, amine, hydrogen or hydroxyl;
in the preparation of an agent for preventing, circumventing or ameliorating resistance of viable human tumour cells to DNA damaging chemotherapeutic agents, to methotrexate or to radiation with the proviso that said nucleoside analogue is not AZT if said DNA damaging chemotherapeutic agent is cisplatin. The halogen is preferably selected from fluorine and iodine.

The invention further provides a use of a nucleoside analogue selected from ddC or AZT together with platinum for the preparation of an agent for preventing, circumventing or ameliorating resistance of viable human tumour cells to DNA damaging chemotherapeutic agents, to methotrexate or to radiation.

According to a further embodiment, the invention provides a liposome having entrapped therein a nucleoside analogue as defined in claim 1 and a DNA damaging agent, for delivery of said nucleoside analogue to a tumour site for preventing, circumventing or ameliorating resistance of viable human tumour cells to DNA damaging chemotherapeutic agents, to methotrexate or to radiation.

An important part of the invention entails a recognition of the interdependence of three factors to achieve amelioration or circumvention of resistance, namely (i) whether the cells are sensitive or resistant, and if resistant, the degree of resistance, (ii) the type of cancer cell involved and (iii) the sequence in which the DNA damaging agent and nucleoside analog are administered. By an appropriate regimen implementing the consequences of such interdependence, resistance to DNA damaging therapy may be effectively treated.

For example, in instances where a specific nucleoside analog may be ineffective with a specific type of cancer resistant cells, pretreatment of such cells with, e.g., cisplatin may result in an effective regimen.

Specific nucleoside analogs useful in the invention include, but are not limited to, 3' azido-2',3' dideoxythymidine (AZT), dideoxy inosine (DDI), each of the purine nucleosides described in United States patent 4,861,759, and Yarchoan, supra, and ganciclovir which, as the triphosphate, is a potent inhibitor of DNA polymerase α.

Table I illustrates the efficacy of ganciclovir as a suicide substrate evidenced by its toxicity to cisplatin resistant and sensitive A2780 cell lines.

**TABLE I**

| Cytotoxic Studies with Ganciclovir in Human Carcinoma Cells Sensitive ("S") and Resistant (DDP) to Cisplatin | | |
|---|---|---|
| Cell Line | αDNA Polymerase Activity* | Ganciclovir EC₅₀ (µM) |
| A2780S | 95 (±6.7) | 110 (±2.1) |
| A2780DDP | 214 (± 8.1) | 30 (±1.5) |
| HCT8S | 30.9 (±4.1) | 60 (±2.3) |
| HCT8DDP | 41.7 (±4.4) | 40 (±4.2) |

| | | |
|---|---|---|
| *The determination of DNA polymerase α was calculated as pmoles/10⁶ cells/10 min (mean ±SD). The A2780 and HCT8 cells were plated in 35 mm petri dishes with RPMI 1640 nutrient. Twenty-four hours later, the cells were treated with 6 concentrations, of ganciclovir in saline. The concentrations were in equal increments from 1 micromolar to 100 micromolar. Six days later the cells were counted on a Coulter Counter. The experiment was done in triplicate. | | |

As indicated in Table I, A2780 cells resistant to cisplatin are 3-4 fold more sensitive to ganciclovir. This correlates with an approximate 2 fold increase in DNA polymerase α levels. A similar but more modest effect is observed for HCT8 cell lines for both DNA polymerase α levels and changes in ganciclovir sensitivity.

Table II reflects inhibition of A2780 cell sensitive (S) and resistant (DDP) growth by Cisplatin AZT Ara A, Ara C, and ddC. The experiment was conducted in triplicate in the manner described with respect to Table I.

**TABLE II**

| Inhibition of A2780 cell growth by cancer chemotherapeutic agents EC₅₀ (µM)^{c} | | | | |
|---|---|---|---|---|
| Compound | Treatment Time (hr) | A2780S | A2780DDP | Ratio^{a} |
| Cisplatin | (1) | 7.0(±1.4)^{b} | 90.0(±2.2)^{b} | +12.8 |
| araA | C.exp.* | 16.2(±2.4)^{b} | 64.7(±6.1)^{b} | + 4.0 |
| araC | C.exp.* | 0.1(±0.01)^{b} | 4.5(±0.5)^{b} | +45.0 |
| ddC | C.exp.* | 1.5(±0.8)^{b} | 4.0(±1.6)^{b} | + 2.6 |

| | | | | |
|---|---|---|---|---|
| ^{a}Ratio of EC₅₀ of sensitive cells to that of resistant cells which denotes the degree of resistance of cross-resistance. | | | | |
| ^{b}Mean ±SD | | | | |
| ^{c}EC₅₀ is the concentration of drug that reduces cell proliferation by 1/2 during 6 days subsequent to an exposure with a cancer chemotherapeutic agent. | | | | |
| *C.exp. indicates continuous exposure. | | | | |

The data in Table II indicates the degree of resistance is reduced by Ara A and ddC alone but increased by Ara C alone.

Table III illustrates the inhibition of A2780 cell growth by sequential treatment first with cisplatin and then with ddC and Ara A.

**Table III**

| Inhibition of A2780 Cell Growth by Cancer Chemotherapeutic Agents. | | |
|---|---|---|
| 1. Sequencing | EC₅₀ (µM)^{b} A2780S | EC₅₀ (µM)^{b} A2780DDP |
| (a) Cisplatin (1 hr), washout, ganciclovir | 64.0 | 30.0 |
| (b) Cisplatin (1 hr), washout, ddC | 0.2(+0.02)^{a} | 3.0(+0.1)^{a} |
| (c) Cisplatin (1 hr), washout, araA | 8.5 | 30.0 |

| | | |
|---|---|---|
| ^{a}Mean+SD | | |
| ^{b}EC₅₀ is the concentration of drug that reduces cell proliferation by 1/2 during 6 days subsequent to an exposure with a cancer chemotherapeutic agent. | | |

A2780 cells were well-plated (35 mm dishes) and 24 hours later the cells were treated with cisplatin for 1 hr., washed out and then treated with 6 concentrations of from 1 µM to 100 µM increased in equal increments. of a nucleoside analog in saline. Cells were then washed and incubated for 6 days. The cells were counted on a Coulter Counter and the experiment was done in triplicate.

As Table III also indicates, synergistic combinations are provided by administration of the nucleoside analogs of the invention sequentially with cisplatin or radiation.

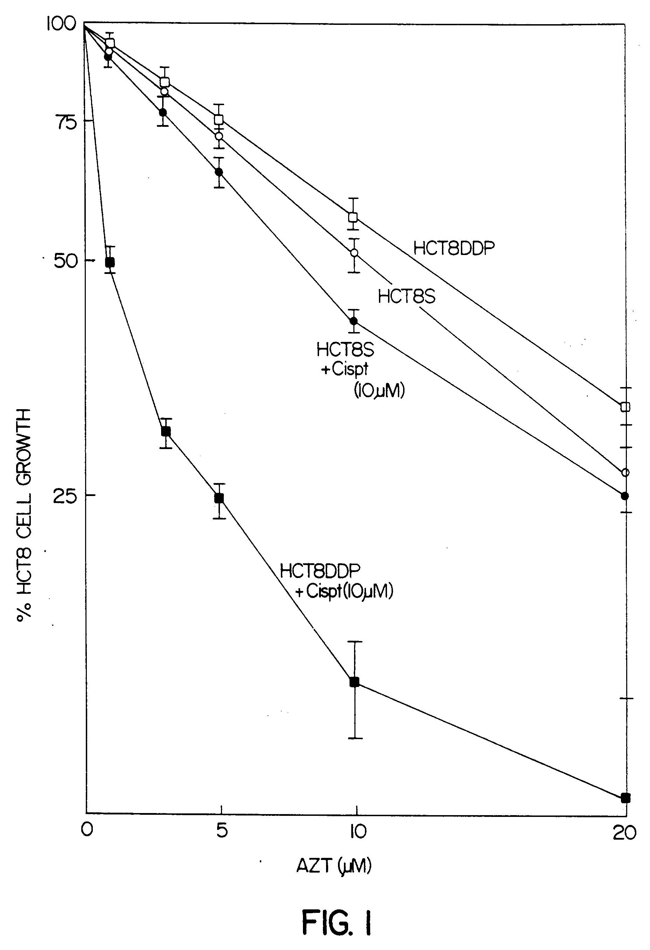

By way of background, cisplatin cells are collaterally resistant to AZT alone and to the TTP antimetabolites. Increased TTP levels in cisplatin resistant cells results in a competitive disadvantage for AZTTP incorporation. To remove DNA adducts, resistant phenotype cells such as HCT8 cells resistant to cisplatin apparently metabolize their DNA more rapidly than sensitive cells. Repair gaps following cisplatin adduct removal provide sites for the incorporation of suicide substrates such as AZTTP. Sequential treatment of cancer cells, first with cisplatin and then AZT, enhances the reduction in cell growth. The exposure first to cisplatin enhances the capacity of the cells to synthesize and repair DNA damage which is then exploited by the administration of the suicide substrate AZTTP. This phenomenon is illustrated by Figure 1.

AZT is a thymidine derivative metabolized via the thymidine metabolic pathway. The triphosphate AZTTP acts as a suicide substrate by causing DNA chain termination. See, White, E.L., Biochem. and Biophys. Res. Comm. 161: 393-398 (1989).

For comparative purposes, the biochemical basis for this selective synergy between cisplatin and AZT, the capacity of A2780DDP cells to repair DNA was quantitated. A dual labeled (¹⁴C/³H) experiment was designed to measure simultaneously DNA synthesis and degradation in response to cisplatin. In Table IV, A2780 cells were incubated for 48 hrs with (¹⁴C) thymidine to label DNA, the A2780 cells were then exposed for 1 hr with (³H) AZT prior to cisplatin (20µM) addition. A2780S cells respond to cisplatin with a modest degradation (10%) of (¹⁴C) DNA over 3 hrs. In contrast, A2780DDP degradation 40.5% of the (¹⁴C) DNA in 3 hrs. A2780S responded to cisplatin with an increased (18.8%) incorporation of (³H) AZT into trichloroacetic acid (TCA) soluble material; in contrast, A2780DDP cells incorporate 40.8% (³H) AZT into TCA insoluble material over 3 hrs. Transplatin at equimolar concentrations (20µM) had no influence on DNA synthesis or degradation in A2780 cells.

**TABLE IV (For Reference)**

| Synthesis and Degradation of DNA in A2780 Cells Treated with Cisplatin | | | | |
|---|---|---|---|---|
| Treatment Time (min) | ¹⁴C-Tdr/DNA | | 3H-AZT/DNA | |
| | A2780S | A2780DDP | A2780S | A2780DDP |
| 0 | 6.49 | 17.78 | 7.79 | 26.96 |
| 45 | 6.21 | 14.44 | 6.27 | 31.62 |
| 90 | 5.71 | 12.59 | 7.23 | 32.31 |
| 180 | 6.06 | 10.57 | 9.25 | 37.96 |

As Table V shows, this phenomenon in A2780DDP cells was not unique to AZT, both ddC and ara A were also incorporated into TCA precipitable material when treated with cisplatin (20µM).

**TABLE V**

| Incorporation of Labelled Nucleoside Analogs into Macromolecules in A2780DDP Cells Treated with Cisplatin | | | | |
|---|---|---|---|---|
| Substrate | After Cisplatin Treatment (min) | | | |
| | 0 | 45′ | 90′ | 180′ |
| (³H)AZT (reference) | 26.96 | 31.62 | 32.31 | 37.96 |
| (³H)araA | 19.01 | 24.21 | 27.91 | 32.70 |
| (³H)ddC | 15.09 | 36.94 | 42.61 | 45.93 |

If the A2780DDP cells were not pretreated with cisplatin, then a low level of (³H) nucleoside analog incorporation in TCA soluble material was detected over three hours and was similar to the value at zero time. Also, the turnover of (¹⁴C) thymidine in DNA was stable in A2780 cells in the absence of cisplatin treatment. Only by the exposure to cisplatin was the DNA turnover increased significantly in A2780DDP cells. After 3 hrs, there were changes in the rate of DNA synthesis and repair in A2780DDP cells only as measured by ¹⁴C and ³H. This may be explained by changes in deoxynucleotide pools.

Any of the nucleoside analogs of this invention can be used in this sequential treatment protocol.

Table VI includes EC₅₀ data further exemplifying the invention. The data reported by Table VI was obtained by substantially the same procedure as that described with respect to Table III. In Table VI, "(C)" indicates continuous exposure, "(1h)" and "(2h)" indicate exposure for 1 hour and 2 hours; "HU" indicates hydroxyurea.

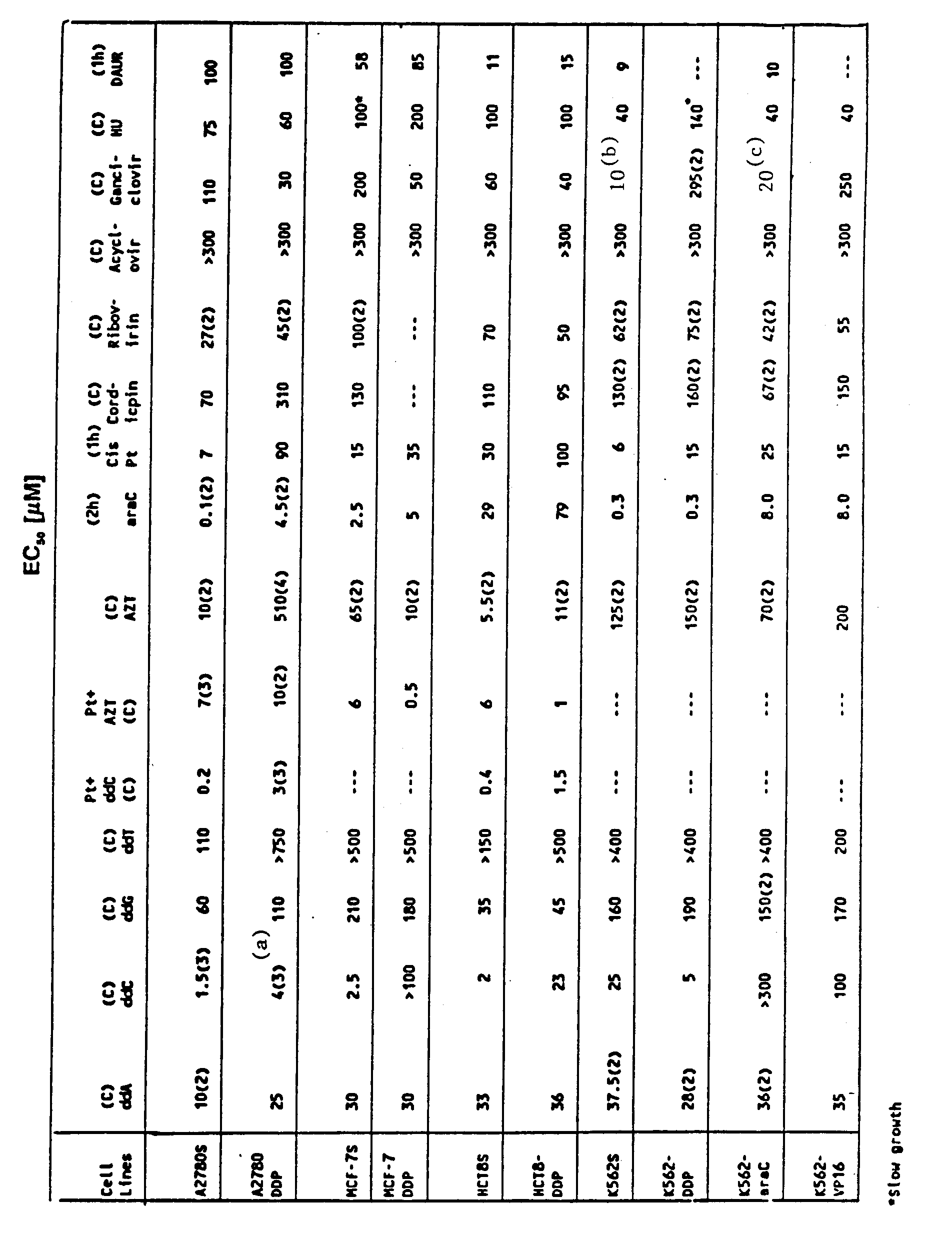

Referring to Table VI:
(a) Figures in parentheses, e.g., (2), indicate replications of experiments.

The data in Table VI indicates, inter alia, the following:
1. A2780:
   Ovarian carcinoma cells become resistant to cisPt, requiring >12 fold more cisPt.
   CisPt resistant cells can be killed with 3.3 fold LOWER ganciclovir concentration (30uM).
   By using cisPt in combination with AZT, both cisPt resistant and sensitive cells can be killed with equal efficiency.
2. MCF7:
   Human breast cancer cells resistant to cisPt (by a factor of 2.3) are 4 fold MORE sensitive to ganciclovir and 6.5 fold more sensitive to AZT.
3. HCT8:
   Human colon cancer cells which have become 2.7 fold resistant to cisPt are 1.5 fold more sensitive to ganciclovir.
4. K562:
   Human leukemia cells resistant to cisPt are more sensitive to ddC by a factor of 5.
   Human leukemia cells resistant to araC are 2 fold more sensitive to cordecepin and AZT.

CEM cells were made 15.8-fold resistant to methotrexate (MTX) by weekly (2 hr) pulses and cloned in soft agar. The optimal growth requirements for the CEMS/S and CEM/MTX for folinic acid cells were 2nM and 10nM. If the cells were grown in higher concentrations of folinic acid (10⁻⁷M), both cell lines were correspondingly more resistant to MTX. In contrast, fluoropyrimidine cytotoxicity was enhanced in both cell lines with increasing concentrations of folinic acid from 10⁻⁷M to 10⁻⁶M. At concentrations of folinic acid that are not optimal for cell growth, both CEM cell lines are hypersensitive to fluoropyrimidines. In addition, CEM/MTX cells are more hypersensitive to fluoropyrimidines than CEM/S cells at (10⁻⁸M) folinic acid or lower concentrations.

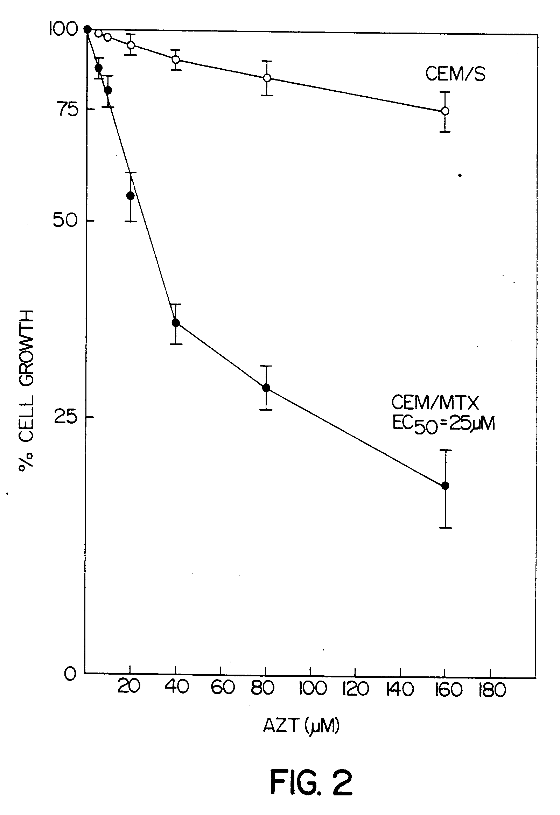

There was an increase in DHFR enzyme activity (11.07-fold) due to an eight-fold increase in DHFR gene amplification and expression in CEM/MTX cells. There was also a corresponding 2-fold increase in thymidine kinase (TK) enzyme activity and TK gene expression in CEM/MTX cells. AZT by continuous exposure (up to 160µM) showed minimal cytotoxicity in CEM/S cells (Figure 2). To obtain the data depicted by Figure 2, CEM/S and CEM/MTX cells were continuously exposed to AZT (1-160µM). CEM cells untreated with nucleoside analog were the controls (100%). The experiment was performed three separate times in duplicate. In contrast, CEM/MTX were collaterally sensitive to AZT (EC₅₀, 25µM). dTMP synthase remained unchanged in both cell lines.

There was no change in dTMP synthase as measured by gene expression or enzyme activity, but the CEM/MTX cells were shown to have 2-fold elevation in TK activity. This may help CEM/MTX cells to salvage more thymidine, creating less dependence on dTMP synthase. Based on this unique property of CEM/MTX cells, preliminary evidence suggests that AZT can be activated via the thymidine metabolic pathway by the increased TK activity in CEM/MTX cells. Higher concentrations of AZTTP could be achieved in CEM/MTX cells, in contrast to CEMS cells, thus AZTTP would act as a more efficient suicide substrate by causing chain termination. Other deoxynucleoside analogs are currently under investigation to further exploit their selectivity against drug resistant cells. The DNA polymerase α and β are not increased in CEM/MTX cells, and this may explain the lack of cross resistance to AZT as seen in cisplatin resistant cells. It would be of interest clinically if MTX resistant cells could be exploited by the currently available nucleoside analogs. Patients that fail MTX treatment could easily be tested by the PCR assay for changes in gene expression of TK.

Table VII reflects data derived from the T-cell lymphocyte leukemia cell line "CEM":

**TABLE VII**

| Nucleoside Analogs Effective Against Human Tumors | | | |
|---|---|---|---|
| | Cell Line | Drug | EC₅₀(µM)* |
| 15. | GEMS | AZT | N.E.** |
| 16. | CEMDDP | AZT | N.E.** |
| 17. | CEMMTX | AZT | 25.0 |

| | | | |
|---|---|---|---|
| * = Continuous exposure. | | | |
| **N.E. = No effect with the nucleoside analogs. | | | |

The treatment of human patients is an important aspect of the invention. Serum levels of nucleoside analogs are achievable to micromolar range which is effective to kill resistant cells. Preferably, an appropriate nucleoside analog is administered intravenously in a therapeutically effective amount, e.g., about 10 mg/kg body weight, while suspended or dissolved in an appropriate carrier such as water.

Nucleoside analogs in combination with a DNA damaging chemotherapeutic agent, may be encapsulated in liposomes for delivery to a tumour site in a patient's body. Such liposomal products may be produced in a known manner. See, generally, United States patents 4,797,285 and 4,873,088, each incorporated herein by reference.

This aspect of the invention, accordingly, includes nucleoside analogs of Formula I and Formula II encapsulated in liposomes with a DNA damaging agent. Such agents include, but are not limited to, cisplatin and methotrexate. More specifically, the invention includes liposomes in which one or more of AZT, ganciclovir, AraA, AraC or ddC is co-entrapped with cisplatin or methotrexate. Although sequential administration of the DNA damaging agent and then of the nucleoside analog is preferred, particularly in human therapy, simultaneous administration is appropriate. It appears that such simultaneous administration results in the DNA damaging agent having the desired effect prior to the time that the nucleoside analog reaches peak effectiveness.

## Claims

1. The use of a nucleoside analogue of formula I or formula II 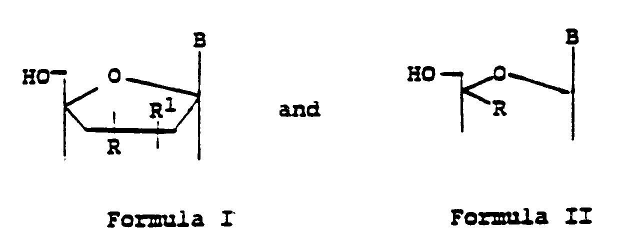 wherein:
B is adenine, thymine, cytosine, guanine, inosine or a heterocyclic compound derived therefrom, for example 5-methylcytosine; and
R and R¹ are, independently, halogen, azide, amine, hydrogen or hydroxyl;
in the preparation of an agent for preventing, circumventing or ameliorating resistance of viable human tumour cells to DNA damaging chemotherapeutic agents, to methotrexate or to radiation with the proviso that said nucleoside analogue is not AZT if said DNA damaging chemotherapeutic agent is cisplatin.

2. The use as claimed in claim 1 wherein the human tumour cells are ovarian tumour cells, HCT8 cells or K562 cells.

3. The use as claimed in any one of claims 1 and 2 wherein the nucleoside analogue is ganciclovir, AraA, AraC, or ddC.

4. The use of a nucleoside analogue selected from ddC or AZT together with platinum for the preparation of an agent for preventing, circumventing or ameliorating resistance of viable human tumour cells to DNA damaging chemotherapeutic agents, to methotrexate or to radiation.

5. A liposome having entrapped therein a nucleoside analogue as defined in claim 1 and a DNA damaging agent, for delivery of said nucleoside analogue to a tumour site for preventing, circumventing or ameliorating resistance of viable human tumour cells to DNA damaging chemotherapeutic agents, to methotrexate or to radiation.

6. A liposome as claimed in claim 5 wherein the DNA damaging agent is cisplatin.

7. A liposome according to claim 5 or 6 wherein said nucleoside analogue is selected from one or more of AZT, ganciclovir, AraA, AraC or ddC.

## Patentansprüche

1. Verwendung eines Nukleosidanalogons der Formel I oder II 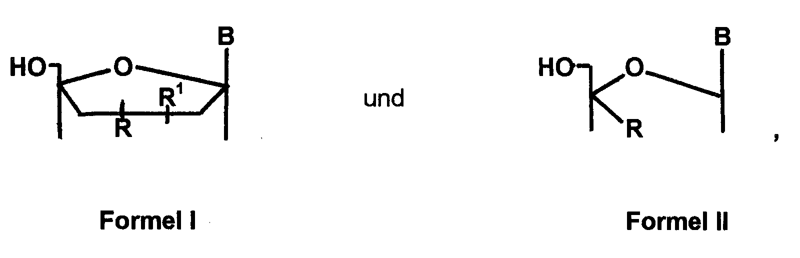 worin:
B Adenin, Thymin, Cytosin, Guanin, Inosin oder eine sich davon ableitende heterocyclische Verbindung, z.B. 5-Methylcytosin, und
R und R¹ unabhängig Halogen, Azid, Amin, Wasserstoff oder Hydroxyl sind,
zur Herstellung eines Mittels zur Hinderung, Beeinflussung oder Verbesserung der Widerstandsfähigkeit lebensfähiger menschlicher Tumorzellen gegen DNA-schädigende chemotherapeutische Mittel, Methotrexat oder Bestrahlung, mit dem Vorbehalt, daß das Nucleosidanalogon nicht AZT ist, wenn das DNA-schädigende chemotherapeutische Mittel Cisplatin ist.

2. Verwendung nach Anspruch 1, worin die menschlichen Tumorzellen Eierstocktumorzellen, HCT8-Zellen oder K562-Zellen sind.

3. Verwendung nach einem der Ansprüche 1 und 2, worin das Nucleosidanalogon Ganciclovir, AraA, AraC, oder ddC ist.

4. Verwendung eines aus ddc oder AZT zusammen mit Platin ausgewählten Nucleosidanalogons zur Herstellung eines Mittels zur Hinderung, Beeinflussung oder Verbesserung der Widerstandsfähigkeit lebensfähiger menschlicher Tumorzellen gegen DNA-schädigende cheomtherapeutische Mittel, Methotrexat oder Strahlung.

5. Ein in ein Liposom eingeschlossenes Nucleosidanalogon gemäß Anspruch 1 und ein DNA-schädigendes Mittel zur Lieferung des Nucleosidanalogons zu einer Tumorstelle zur Hinderung, Beeinflussung oder Verbesserung der Widerstandsfähigkeit lebensfähiger menschlicher Tumorzellen gegen DNA-schädigende chemotherapeutische Mittel, Methotrexat oder Strahlung.

6. Liposom nach Anspruch 5, worin das DNA-schädigende Mittel Cisplatin ist.

7. Liposom nach Anspruch 5 oder 6, worin das Nucleosidanalogon aus einem oder mehreren von AZT, Ganciclovir, AraA, AraC oder ddC ausgewählt ist.

## Revendications

1. L'utilisation d'un analogue de nucléoside de formule I ou de formule II 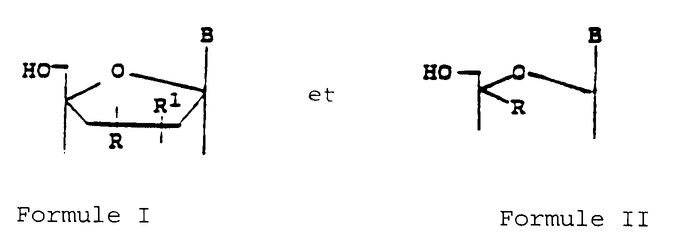 où
B est l'adénine, la thymine, la cytosine, la guanine, l'inosine ou un composé hétérocyclique qui en est dérivé, par exemple la 5-méthylcytosine ; et
R et R¹ sont indépendamment un halogène, un azide, une amine, l'hydrogène ou un groupe hydroxyle ;
dans la préparation d'un agent destiné à prévenir, éviter ou rendre plus tolérable la résistance de cellules tumorales humaines viables aux agents chimiothérapiques altérant l'ADN, au méthotrexate ou aux radiations, avec la condition que ledit analogue de nucléoside ne soit pas l'AZT si ledit agent chimiothérapique altérant l'ADN est le cisplatine.

2. L'utilisation telle que revendiquée dans la revendication 1, dans laquelle les cellules tumorales humaines sont des cellules tumorales ovariennes, des cellules HCT8 ou des cellules K562.

3. L'utilisation telle que revendiquée dans l'une quelconque des revendications 1 et 2, dans laquelle l'analogue de nucléoside est le ganciclovir, AraA, AraC ou ddC.

4. L'utilisation d'un analogue de nucléoside choisi parmi la ddC ou l'AZT en association avec du platine pour la préparation d'un agent destiné à prévenir, éviter ou rendre plus tolérable la résistance des cellules tumorales humaines viables aux agents chimiothérapiques altérant l'ADN, au méthotrexate ou aux radiations.

5. Un liposome qui renferme un analogue de nucléoside tel que défini dans la revendication 1 et un agent altérant l'ADN, destiné à délivrer ledit analogue de nucléoside à un site tumoral pour prévenir, éviter ou rendre plus tolérable la résistance de cellules tumorales humaines viables aux agents chimiothérapiques altérant l'ADN, au méthotrexate ou aux radiations.

6. Un liposome tel que revendiqué dans la revendication 5, dans lequel l'agent altérant l'ADN est le cisplatine.

7. Un liposome selon la revendication 5 ou 6, dans lequel ledit analogue de nucléoside est choisi parmi un ou plusieurs de AZT, ganciclovir, AraA, AraC ou ddC.
